# EUROPEAN PATENT APPLICATION

(11) **EP 2 314 292 A1**
(43) Date of publication of application: **27.04.2011**
(21) Application number: 09173525.8
(22) Date of filing: 20.10.2009
(51) Int. Cl.: A61K 31/277, A61K 31/55, A61P 33/00, A61P 33/10

(54) **Endoparasiticidal compositions**

(71) Applicant: Novartis AG, 4056 Basel (CH)
(72) Inventor: Kaminsky, Ronald, 1789Lugnorre (CH); Little, Andrew, 4002 Basel (CH); Redpath, Arthur, 4114 Hofstetten (CH); Schorderet Weber, Sandra, 2000 Neuchâtel (CH)
(74) Representative: Liphardt, Bernd

(57) **Abstract**

The invention relates to a composition for controlling endoparasites in and on animals, which comprises a combination of
(A) a compound of formula (I) 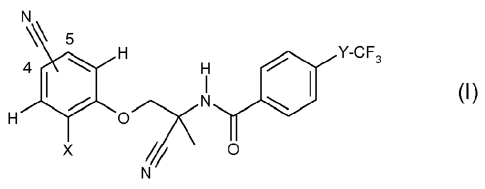 and
(B) paraherquamide A or a derivative or analogue thereof,
wherein X and Y are as defined in the claims. The compositions are useful in the control of endoparasites, in particular helminths, in and on warm-blooded animals.

## Description

The present invention relates to novel endoparasiticidal compositions and their use in the control of endoparasites, in particular helminths.

There is a continuing need for new agents to manage endoparasitic infestations in livestock animals due to the increasing prevalence of parasites, in particular nematodes, that are resistant to many of the agents currently approved for this indication.

Recently, the chemical class of aminoacetonitrile derivatives (AAD) has been found to be extremely active against endoparasites such as helminths. For example, WO 2005/44784 discloses specific novel members of this chemical class, which offer a new route of combating endoparasitic infestations in livestock animals.

It now has been found, that the combination of an anthelmintic of the class of aminoacetonitrile derivatives with a derivative of the paraherquamide/marcfortine class offers further unexpected advantages. For example, said combination of the two active ingredients broadens the activity spectrum with regard to the endo-parasites in a synergistic manner. Moreover, the number of treatments per year may be reduced significantly. In addition, the pest resistance management is improved, meaning that the occurrence of resistance against one active ingredient is delayed drastically by the administration of the combination product instead of applying one active ingredient only. Another advantage is that the combination product can be used successfully even in those cases where the worm population has already developed resistance against common anthelmintics.

According, the present invention in one aspect relates to a composition for controlling endoparasites in and on animals, which comprises a combination, in variable proportions, of
(A) a compound of formula (1) 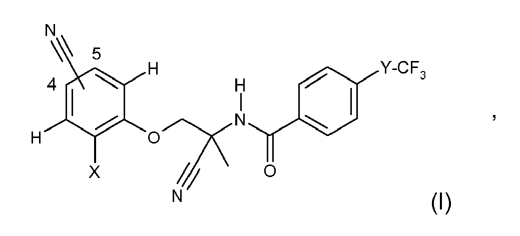 wherein
   X signifies Cl, Br or CF₃, and
   Y signifies a single bond, O, S, S(O) or S(O)₂; and
(B) paraherquamide A or a derivative or analogue thereof.

The compounds (A) and their synthesis are known, for example, from WO 2005/44784. The compounds of the present invention have an asymmetric carbon atom in the 1-position labelled with (1*) in the formula I' below 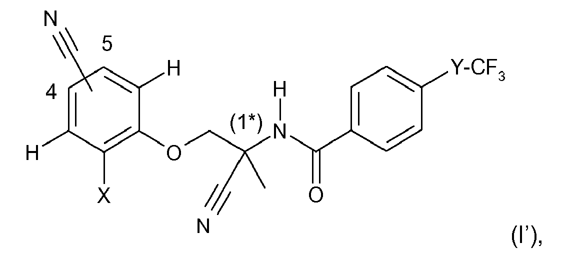 wherein X and Y are as defined above. Accordingly, the compounds of formula I may exist as optical isomers. The present invention includes individual enantiomers of the compounds of formula I and mixtures thereof, including racemates. In addition, it has been found that following the separation of the racemates into the two pure enantiomers by standard methods, e. g. by chemical resolution using optically active acid or base or by chromatography on chiral adsorbents, e.g. high-pressure liquid chromatography on acetyl cellulose, or by the process as disclosed in WO 2006/50887, one of them has proven to be biologically less active (the distomer), whereas the other enantiomer is highly bioactive (the eutomer). In general, the (1S)-enantiomers of the formula (I') are highly bioactive, whereas the (1 R)-enantiomers are less bioactive.

The compounds of formula I may exist as geometric isomers. The present invention includes such compounds in the cis (Z-) or trans (E-) configuration, as well as mixtures of these geometric isomers.

The compounds of formula I may exist in more than one tautomeric form. The present invention encompasses all tautomers, as well as mixtures thereof.

Certain compounds of formula I may be able to form salts with acids or bases. The present invention includes said compounds of formula I in form of a salt to the extent that they are pharmaceutically or veterinarily acceptable.

The compounds of formula I and their salts may exist in unsolvated or solvated forms. The term solvate herein describes a molecular complex comprising the compound of formula I and one or more pharmaceutically or veterinarily acceptable solvents, for example ethanol or water. In case of water. The term "hydrate" is used.

Preferred embodiments of the compounds of formula (I) are as follows:
(A1) A compound of formula I, wherein X is Cl or CF₃, in particular CF₃.
(A2) A compound of formula I, wherein Y is S, S(O) or S(O₂); and X signifies Cl, Br or CF₃, in particular Cl or CF₃, and especially CF₃.
(A3) A compound of formula I, wherein Y is S or S(O₂); and X signifies Cl, Br or CF₃, in particular Cl or CF₃, and especially CF₃.
(A4) A compound of formula I, selected from the group consisting of
   N-(1-cyano-2-(4-cyano-2-chlorophenoxy)-1-methylethyl)-4-trifluoromethylbenzamide;
   N-(1-cyano-2-(4-cyano-2-bromophenoxy)-1-methylethyl)-4-trifluoromethylbenzamide;
   N-(1-cyano-2-(4-cyano-2-trifluoromethylphenoxy)-1-methylethyl)-4-trifluoromethyl-benzamide;
   N-(1-cyano-2-(5-cyano-2-chlorophenoxy)-1-methylethyl)-4-trifluoromethylbenzamide;
   N-(1-cyano-2-(5-cyano-2-bromophenoxy)-1-methylethyl)-4-trifluoromethylbenzamide; and
   N-[1-cyano-2-(5-cyano-2-trifluoromethyl phenoxy)-1-methylethyl)-4-trifluoromethyl-benzamide.
(A5) A compound of formula I, selected from the group consisting of
   N-(1-cyano-2-(4-cyano-2-ch lorophenoxy)-1-methylethyl)-4-trifluoromethoxybenzamide;
   N-(1-cyano-2-(4-cyano-2-bromophenoxy)-1-methylethyl)-4-trifluoromethoxybenzamide;
   N-(1-cyano-2-(4-cyano-2-trifluoromethylphenoxy)-1-methylethyl)-4-trifluoromethoxy-benzamide;
   N-(1-cyano-2-(5-cyano-2-chlorophenoxy)-1-methylethyl)-4-trifluoromethoxybenzamide;
   N-(1-cyano-2-(5-cyano-2-bromophenoxy)-1-methylethyl)-4-trifluoromethoxybenzamide; and
   N-(1-cyano-2-(5-cyano-2-trifluoromethylphenoxy)-1-methylethyl)-4-trifluoromethoxy-benzamide.
(A6) A compound of formula I, selected from the group consisting of
   N-(1-cyano-2-(4-cyano-2-chlorophenoxy)-1-methylethyl)-4-trifluoromethylsulfanylbenzamide;
   N-(1-cyano-2-(4-cyano-2-bromophenoxy)-1-methylethyl)-4-trifluoromethylsulfanyl-benzamide;
   N-(1-cyano-2-(4-cyano-2-trifluoromethylphenoxy)-1-methylethyl)-4-trifluoromethylsulfanyl-benzamide;
   N-(1-cyano-2-(5-cyano-2-chlorophenoxy)-1-methylethyl)-4-trifluoromethylsulfanylbenzamide;
   N-(1-cyano-2-(5-cyano-2-bromophenoxy)-1-methylethyl)-4-trifluoromethylsulfanyl-benzamide;
   N-(1-cyano-2-(5-cyano-2-trifluoromethylphenoxy)-1-methylethyl)-4-trifluoromethylsulfanyl-benzamide;
   N-(1-cyano-2-(4-cyano-2-chlorophenoxy)-1-methylethyl)-4-trifluoromethylsulfinylbenzamide;
   N-(1-cyano-2-(4-cyano-2-bromophenoxy)-1-methylethyl)-4-trifluoromethylsulfinylbenzamide;
   N-(1-cyano-2-(4-cyano-2-trifluoromethylphenoxy)-1-methylethyl]-4-trifluoromethylsulfinyl-benzamide;
   N-(1-cyano-2-(5-cyano-2-chlorophenoxy)-1-methylethyl)-4-trifluoromethylsulfinylbenzamide;
   N-(1-cyano-2-(5-cyano-2-bromophenoxy)-1-methylethyl)-4-trifluoromethylsulfinylbenzam ide;
   N-(1-cyano-2-(5-cyano-2-trifluoromethylphenoxy)-1-methylethyl)-4-trifluoromethylsulfinyl-benzamide;
   N-(1-cyano-2-(4-cyano-2-chlorophenoxy)-1-methylethyl)-4-trifluoromethylsulfonylbenzamide;
   N-(1-cyano-2-(4-cyano-2-bromophenoxy)-1-methylethyl)-4-trifluoromethylsulfonyl-benzamide;
   N-(1-cyano-2-(4-cyano-2-trifluoromethylphenoxy)-1-methylethyl]-4-trifluoromethylsulfonyl-benzamide;
   N-(1-cyano-2-(5-cyano-2-chlorophenoxy)-1-methylethyl)-4-trifluoromethylsulfonyl-benzamide;
   N-(1-cyano-2-(5-cyano-2-bromophenoxy)-1-methylethyl)-4-trifluoromethylsulfonyl-benzamide; and
   N-(1-cyano-2-(5-cyano-2-trifluoromethylphenoxy)-1-methylethyl)-4-trifluoromethylsulfonyl-benzamide.
(A7) The compound N-(1-cyano-2-(5-cyano-2-trifluoromethylphenoxy)-1-methylethyl)-4-trifluoromethylsulfanylbenzamide.
(A8) The (1S)-enantiomer of each of the compounds mentioned in (A1) - (A7).
(A9) The enantiomer N-[(1S)-1-cyano-2-(5-cyano-2-trifluoromethylphenoxy)-1-methylethyl]-4-trifluoromethylsulfanylbenzamide.

Further valuable aminoacetonitrile compounds to be combined with the paraherquamide derivative (B) are:
(A10) N-[1-cyano-2-(5-cyano-2-trifluoromethylphenoxy)-1-methylethyl]-4-pentafluorothio-benzamide;
(A11) N-[(1S)-(1-cyano-2-(5-cyano-2-trifluoromethylphenoxy)-1-methylethyl]-4-pentafluorothiobenzamide;
(A12) N-[1-cyano-2-(4-cyano-2-trifluoromethylphenoxy)-1-methylethyl]-4-pentafluorothiobenzamide;
(A13) N-[(1 S)-(1-cyano-2-(4-cyano-2-trifluoromethylphenoxy)-1-methylethyl]-4-pentafluoro-thiobenzamide;
(A14) N-[1-cyano-2-(5-cyano-2-chlorophenoxy)-1-methylethyl]-4-pentafluorothiobenzamide;
(A15) N-[(1S)-(1-cyano-2-(5-cyano-2-chlorophenoxy)-1-methylethyl]-4-pentafluorothiobenzamide;
(A16) N-[1-cyano-2-(5-cyano-2-chloro-3-trifluoromethylphenoxy)-1-methylethyl]-4-pentafluorothiobenzamide;
(A17) N-[(1S)-(1-cyano-2-(5-cyano-2-chloro-3-trifluoromethylphenoxy)-1-methylethyl]-4-pentafluorothiobenzamide;
(A18) N-[1-cyano-2-(4,5-dicyano-2-chloro-phenoxy)-1-methylethyl]-4-pentafluorothiobenzamide;
(A19) N-[(1S)-(1-cyano-2-(4,5-dicyano-2-chloro-phenoxy)-1-methylethyl]-4-pentafluorothiobenzamide.

Component (B) of the compositions of the present invention is paraherquamide A or a derivative or analogue thereof.

Paraherquamide A, CAS Registry Number 77392-58-6, has the structure of formula (II) 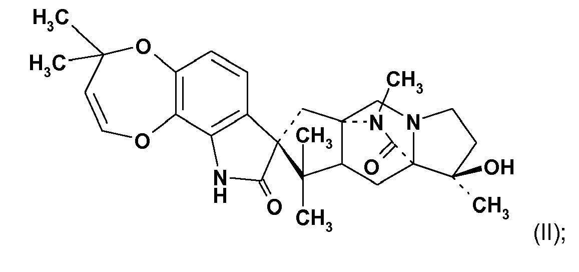 and the chemical name: [1'R-(1'α,5'aβ,7'β,8'aβ,9'aβ)]-2',3',8'a,9'-tetrahydro-1'-hydroxy-1',4,4,8',8',11'-hexamethylspiro[4*H*,8*H*-[1,4]dioxepino[2,3-g]indole-8,7'(8'*H*)-[5*H*,6*H-*5a,9a](iminomethano)[1*H*]cyclopent[*f*]indolizine]-9,10'(10*H*)-dione.

Paraherquamide A is commercially available; the compound may be isolated, for example, as a fungal metabolite of Penicillium paraherquei, now usually called Penicillium brasilianum, using standard fermentation and isolation techniques. Further Penicillium species have been described to produce the compound.

Suitable derivatives of paraherquamide A useful in the compositions of the present invention are, for example,
(B1) dihydroparaherquamide, the compound of formula 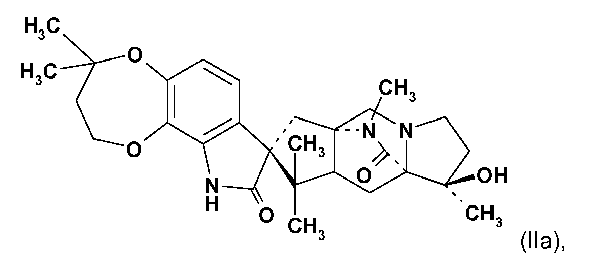 obtainable from paraherquamide A, for example, by catalytic hydrogenation over palladium on a carbon support, as disclosed, for example, in EP-A-31742 A; or
(B2) 2-deoxoparaherquamide, the compound of formula 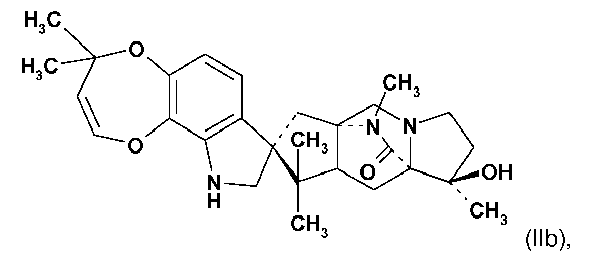 which has the chemical name derquantel or (1'R-(1'α,5'aβ,7'β,8'aβ,9'aβ)]-2',3',8'a,9,9',10-hexahydro-1'-hydroxy-1',4,4,8',8',11'-hexamethylspiro[4*H*,8*H*-[1,4]dioxepino[2,3-g]indole-8,7'(8'*H*)-[5*H*,6*H*-5a,9a](iminomethano)[1*H*]cyclopent[*f*]indolizine]-10'-one. 2-deoxoparaherquamide is known, for example, from US 5,750,695, and may be obtained according to the processes described therein.

Suitable analogues of paraherquamide A are, for example, the marcfortines; Examples are Marcfortine A, known from J.Chem.Soc. Chem. Commun. 1980, 601-602, or Marcfortines B and C, known from Tetrahedron Letters 22, 1977-1980 (1981).

Particularly preferred compositions according to the present invention comprise:
(i) a compound according to formula (I) above and paraherquamide A;
(ii) a compound according to formula (I) above and 2-deoxoparaherquamide;
(iii) the (1S)-enantiomer of a compound of formula (I*) above and paraherquamide A;
(iv) the (1S)-enantiomer of a compound of formula (I*) above and 2-deoxoparaherquamide.
(v) the compound N-(1-cyano-2-(5-cyano-2-trifluoromethylphenoxy)-1-methylethyl)-4-trifluoromethylsulfanylbenzamide and paraherquamide A;
(vi)) the compound N-(1-cyano-2-(5-cyano-2-trifluoromethylphenoxy)-1-methylethyl)-4-trifluoromethylsulfanylbenzamide and 2-deoxoparaherquamide.
vii) the compound N-((1S)-1-cyano-2-(5-cyano-2-trifiuoromethylphenoxy)-1-methylethyl)-4-trifluoromethylsulfanylbenzamide and paraherquamide A;
(viii)) the compound N-((1S)-1-cyano-2-(5-cyano-2-trifluoromethylphenoxy)-1-methylethyl)-4-trifluoromethylsulfanylbenzamide and 2-deoxoparaherquamide.
(ix) the compound N-[1-cyano-2-(4-cyano-2-trifluoromethylphenoxy)-1-methylethyl]-4-pentafluorothiobenzamide and paraherquamide A;
(x) the compound N-[(1S)-(1-cyano-2-(4-cyano-2-trifluoromethylphenoxy)-1-methylethyl]-4-pentafluorothiobenzamide and 2-deoxoparaherquamide;
(xi) the compound N-[1-cyano-2-(5-cyano-2-chlorophenoxy)-1-methylethyl]-4-pentafluorothiobenzamide and paraherquamide A;
(xii) the compound N-[(1S)-(1-cyano-2-(5-cyano-2-chlorophenoxy)-1-methylethyl]-4-pentafluorothiobenzamide and 2-deoxoparaherquamide.

The compositions of the present invention may comprise component (A) and component (B) in any suitable weight ratio, for example in a ratio between 1:100 and 100:1 weight by weight, preferably in a ratio between 1:10 and 10:1 weight by weight, more preferably a ratio between 1:5 and 5:1 weight by weight, and most preferably from 1:3 to 3:1 weight by weight.

Both the aminoacetonitrile of formula (I) and the paraherquamide (B) have an excellent human and animal safety and toxicological profile.

The compositions according to the invention are notable for their broad activity spectrum and are valuable active ingredients for use in pest control, including in particular the control of endoparasites, especially helminths, in and on warm-blooded animals, especially livestock and domestic animals, whilst being well-tolerated by warm-blooded animals and fish.

The compositions of the present invention are effective against helminths, in which the endoparasitic nematodes and trematodes may be the cause of serious diseases of mammals and poultry, e.g. sheep, pigs, goats, cattle, horses, donkeys, dogs, cats, guineapigs and exotic birds. Typical nematodes of this indication are: *Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris* and *Parascaris.* The trematodes include, in particular, the family of *Fasciolideae,* especially *Fasciola hepatica.*

It could also be shown surprisingly and unexpectedly that the compositions of the present invention have exceptionally high efficacy against nematodes that are resistant to many active substances. This can be demonstrated *in vitro* by the LDA test and *in vivo* for example in Mongolian gerbils and sheep. It was shown that amounts of active substance which kill sensitive strains of *Haemonchus contortus* or *Trichostrongylus colubriformis,* are also sufficiently effective at controlling corresponding strains that are resistant to benzimidazoles, levamisol and macrocyclic lactones (for example ivermectin).

Certain pests of the species *Nematodirus, Cooperia* and *Oesophagostonum* infest the intestinal tract of the host animal, while others of the species *Haemonchus* and *Ostertagia* are parasitic in the stomach and those of the species *Dictyocaulus* are parasitic in the lung tissue. Parasites of the families *Filariidae* and *Setariidae* may be found in the internal cell tissue and in the organs, e.g. the heart, the blood vessels, the lymph vessels and the subcutaneous tissue. A particularly notable parasite is the heartworm of the dog, *Dirofilaria immitis.* The compounds of formula I are highly effective against these parasites.

The pests which may be controlled by the compounds of formula I also include those from the class of Cestoda (tapeworms), e.g. the families Mesocestoidae, especially of the genus *Mesocestoides,* in particular *M*. *lineatus; Dipylidiidae,* especially *Dipylidium caninum, Joyeuxiella spp.,* in particular *Joyeuxiella pasquali,* and *Diplopylidium spp.,* and *Taeniidae,* especially *Taenia pisiformis, Taenia cervi, Taenia ovis, Taeneia hydatigena, Taenia multiceps, Taenia taeniaeformis, Taenia serialis,* and *Echinococcus spp.,* most preferably *Taneia hydatigena, Taenia ovis, Taenia multiceps, Taenia serialis; Echinococcus granulosus* and *Echinococcus multilocularis.*

Furthermore, the compositions of the present invention are suitable for the control of human pathogenic parasites. Of these, typical representatives that appear in the digestive tract are those of the genus *Ancylostoma, Necator, Ascaris, Strongyloides, Trichinella, Capillaria, Trichuris* and *Enterobius.* The compounds of the present invention are also effective against parasites of the genus *Wuchereria, Brugia, Onchocerca* and Loa from the family of *Filariidae,* which appear in the blood, in the tissue and in various organs, and also against *Dracunculus* and parasites of the genus *Strongyloides* and *Trichinella,* which infect the gastrointestinal tract in particular.

The good endoparasiticidal activity of the compositions of the present invention corresponds to a mortality rate of at least 50-60% of the endoparasites mentioned. In particular, the compositions of the present invention are notable for the exceptionally long duration of efficacy.

The compositions of the present invention are preferably employed in unmodified form or preferably together with the adjuvants conventionally used in the art of formulation and may therefore be processed in a known manner to give, for example, emulsifiable concentrates, directly dilutable solutions, dilute emulsions, soluble powders, granules or microencapsulations in polymeric substances. As with the compositions, the methods of application are selected in accordance with the intended objectives and the prevailing circumstances.

The formulation, i.e. the agents, preparations or compositions containing the active ingredients (A) and (B) and optionally a solid or liquid adjuvant, are produced in a manner known *per se,* for example by intimately mixing and/or grinding the active ingredients with spreading compositions, for example with solvents, solid carriers, and optionally surface-active compounds (surfactants).

The solvents in question may be: alcohols, such as ethanol, propanol or butanol, and glycols and their ethers and esters, such as propylene glycol, dipropylene glycol ether, ethylene glycol, ethylene glycol monomethyl or -ethyl ether, ketones, such as cyclohexanone, isophorone or diacetanol alcohol, strong polar solvents, such as N-methyl-2-pyrrolidone, dimethyl sulfoxide or dimethylformamide, or water, vegetable oils, such as rape, castor, coconut, or soybean oil, and also, if appropriate, silicone oils.

Preferred application forms for usage on warm-blooded animals in the control of helminths include solutions, emulsions, suspensions (drenches), food additives, powders, tablets including effervescent tablets, boli, capsules, micro-capsules and pour-on formulations, whereby the physiological compatibility of the formulation excipients must be taken into consideration.

The binders for tablets and boli may be chemically modified polymeric natural substances that are soluble in water or in alcohol, such as starch, cellulose or protein derivatives (e.g. methyl cellulose, carboxymethyl cellulose, ethylhydroxyethyl cellulose, proteins such as zein, gelatin and the like), as well as synthetic polymers, such as polyvinyl alcohol, polyvinyl pyrrolidone etc. The tablets also contain fillers (e.g. starch, microcrystalline cellulose, sugar, lactose etc.), glidants and disintegrants.

If the anthelminthics are present in the form of feed concentrates, then the carriers used are e.g. performance feeds, feed grain or protein concentrates. Such feed concentrates or compositions may contain, apart from the active ingredients, also additives, vitamins, antibiotics, chemotherapeutics or other pesticides, primarily bacteriostats, fungistats, coccidiostats, or even hormone preparations, substances having anabolic action or substances which promote growth, which affect the quality of meat of animals for slaughter or which are beneficial to the organism in another way. If the compositions or the active ingredients (A) and (B) contained therein are added directly to feed or to the drinking troughs, then the formulated feed or drink contains the active ingredients preferably in a concentration of ca. 0.0005 to 0.02 % by weight (5-200 ppm).

As a rule, the anthelminthic compositions according to the invention contain 0.1 to 99 % by weight, especially 0.1 to 95 % by weight of active ingredients (A) and (B), 99.9 to 1 % by weight, especially 99.8 to 5 % by weight of a solid or liquid admixture, including 0 to 25 % by weight, especially 0.1 to 25 % by weight of a surfactant.

Application of the compositions according to the invention to the animals to be treated may take place, for example, topically, perorally, parenterally or subcutaneously, the composition being present, for example, in the form of a solution, emulsion, suspension, (drench), powder, tablet, boli, capsule or pour-on- or spot-on formulation. A preferred embodiment of the invention relates to compositions for parenteral use or, in particular, for peroral use.

The pour-on or spot-on method consists in applying the compositions of the present invention to a specific location of the skin or coat, advantageously to the neck or backbone of the animal. This takes place e.g. by applying a swab or spray of the pour-on or spot-on formulation to a relatively small area of the coat, from where the active substance is dispersed almost automatically over wide areas of the fur owing to the spreading nature of the components in the formulation and assisted by the animal's movements.

Pour-on or spot-on formulations suitably contain carriers, which promote rapid dispersement over the skin surface or in the coat of the host animal, and are generally regarded as spreading oils. Suitable carriers are e.g. oily solutions; alcoholic and isopropanolic solutions such as solutions of 2-octyldodecanol or oleyl alcohol; solutions in esters of monocarboxylic acids, such as isopropyl myristate, isopropyl palmitate, lauric acid oxalate, oleic acid oleyl ester, oleic acid decyl ester, hexyl laurate, oleyl oleate, decyl oleate, capric acid esters of saturated fat alcohols of chain length C₁₂-C₁₈; solutions of esters of dicarboxylic acids, such as dibutyl phthalate, diisopropyl isophthalate, adipic acid diisopropyl ester, di-n-butyl adipate or also solutions of esters of aliphatic acids, e.g. glycols. It may be advantageous for a dispersing agent to be additionally present, such as one known from the pharmaceutical or cosmetic industry. Examples are 2-pyrrolidone, 2-(N-alkyl)pyrrolidone, acetone, polyethylene glycol and the ethers and esters thereof, propylene glycol or synthetic triglycerides.

The oily solutions include e.g. vegetable oils such as olive oil, groundnut oil, sesame oil, pine oil, linseed oil or castor oil. The vegetable oils may also be present in epoxidised form. Paraffins and silicone oils may also be used.

A pour-on or spot-on formulation generally contains 1 to 20 % by weight of a compounds (A) and (B), 0.1 to 50 % by weight of dispersing agent and 45 to 98.9 % by weight of solvent.

The pour-on or spot-on method is especially advantageous for use on herd animals such as cattle, horses, sheep or pigs, in which it is difficult or time-consuming to treat all the animals orally or by injection. Because of its simplicity, this method can of course also be used for all other animals, including individual domestic animals or pets, and is greatly favoured by the keepers of the animals, as it can often be carried out without the specialist presence of the veterinarian.

Whereas it is preferred to formulate commercial products as concentrates, the end user will normally use dilute formulations.

Such compositions may also contain further additives, such as stabilisers, anti-foaming agents, viscosity regulators, binding agents or tackifiers, as well as other active ingredients, in order to achieve special effects.

Anthelminthic compositions of this type, which are used by the end user, similarly form a constituent of the present invention.

In each of the processes according to the invention for pest control or in each of the pest control compositions according to the invention, the active ingredients of formula I can be used in all of their steric configurations or in mixtures thereof.

The invention also includes a method of prophylactically protecting warm-blooded animals, especially productive livestock, domestic animals and pets, against parasitic helminths, which is characterised in that the active ingredients of the formula or the active ingredient formulations prepared therefrom are administered to the animals as an additive to the feed, or to the drinks or also in solid or liquid form, orally or by injection or parenterally. The invention also includes the compounds of formula I according to the invention for usage in one of the said processes.

The following Examples illustrate the invention further.

### Preparation of an oral formulation comprising N-((1S)-1-cyano-2-(5-cyano-2-trifluoromethyl-phenoxy)-1-methylethyl)-4-trifluoromethylsulfanylbenzamide (monepantel) and paraherquamide A

Solutions for oral application are prepared by dissolving the active ingredients as outlined in the table below in a 2:1 w/w mixture of DMSO and polyethylenglycol 300 with stirring at room temperature:

**Oral application in DMSO/PEG (2:1) at 5ml/kg**

| No | Compound | Dose mg/kg |
|---|---|---|
| 1a | Monepantel | 0.2 |
| 1b | Monepantel | 0.1 |
| 2a | Paraherquamide A | 0.4 |
| 2b | Paraherquamide A | 0.32 |
| 2c | Paraherquamide A | 0.2 |
| 3 | Monepantel & paraherquamide A | 0.1 & 0.2 |
| 4 | Monepantel & paraherquamide A | 0.1 & 0.32 |
| 5 | Monepantel & paraherquamide A | 0.2 & 0.2 |
| 6 | Monepantel & paraherquamide A | 0.2 & 0.32 |

### Biological Examples:

### In-vivo test on Trichostrongylus colubriformis on Mongolian gerbils (Meriones unguiculatus) using peroral application

Six to eight week old Mongolian gerbils are infected by gavage with ca. 2000 third instar larvae each of *T. colubriformis.* 6 days after infection, the gerbils are treated by peroral application with the test solutions 1a to 6 above in the quantities given in the Table above. Three days after treatment, when most of the *T. colubriformis* are immature adults, the gerbils euthanized and dissected to recover and count the worms. The efficacy is calculated as the % reduction of the number of worms in each gerbil, compared with the geometric average of number of worms from six infected and untreated gerbils.

In this test, the reduction in nematode infestation achieved with solutions 3-6 is in each case considerably higher than that obtained with solutions 1a, 1b, 2a, 2b or 2c alone. In addition, the worm reduction achieved with solution 3 clearly exceeds the combined result of solutions 1b and 2c; the same is true for solutions 4, 5 and 6 relative to the combined result of solutions 1b and 2b, 1a and 2c, and 1a and 2b, respectively.

## Claims

1. A composition for controlling endoparasites in and on animals, which comprises a combination of
(A) a compound of formula (I) 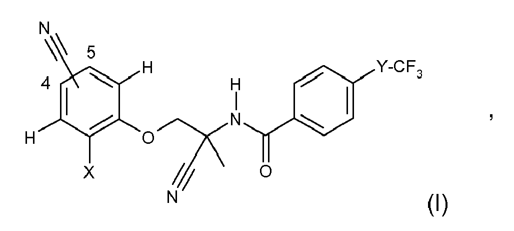 wherein
X signifies Cl, Br or CF₃, and
Y signifies a single bond, O, S, S(O) or S(O)₂; and
(B) paraherquamide A or a derivative or analogue thereof.

2. A composition according to claim 1, wherein component (A) is the (1S)-enantiomer of the compound of formula I' 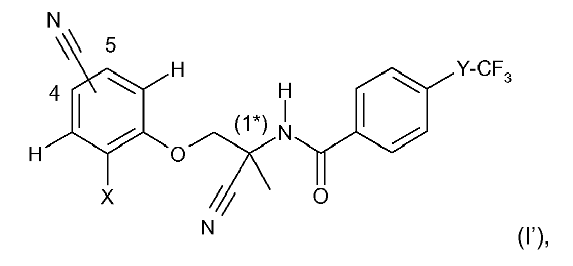 wherein X and Y are as defined in claim 1.

3. A composition according to claim 1, wherein component (A) is the compound N-(1-cyano-2-(5-cyano-2-trifluoromethylphenoxy)-1-methylethyl)-4-trifluoromethylsulfanylbenzamide, in particular (N-[(1S)-1-cyano-2-(5-cyano-2-trifluoromethylphenoxy)-1-methylethyl]-4-trifluoro-methylsulfanylbenzamide.

4. A composition according to any one of claims 1 to 3, wherein component (B) is paraherquamide A.

5. A composition according to any one of claims 1 to 3, wherein component (B) is derquantel (2-deoxoparaherquamide A).

6. A composition according to any one of claims 1 to 5, comprising the components (A) and (B) in a ratio between 10:1 and 1:10 weight by weight, and preferably in a ratio between 1:5 and 5:1 weight by weight.

7. A composition according to any one of claims 1 to 4, which is for peroral or parenteral use.

8. Method of controlling endoparasites in and on warm-blooded animals, which comprises applying to the animals a pharmaceutical effective amount of a composition of any one of claims 1 to 7.

9. Use of a composition according to of any one of claims 1 to 7 in the control of endoparasites, in particular helminths.

10. Use of a composition of any one of claims 1 to 7 in a process for controlling endoparasites, in particular helminths, in and on warm-blooded animals.

11. Use of a composition of any one of claims 1 to 7 in the preparation of a pharmaceutical composition against endoparasites, in particular helminths, in and on warm-blooded animals.
